# EUROPEAN PATENT APPLICATION

(11) **EP 2 878 266 A1**
(43) Date of publication of application: **03.06.2015**
(21) Application number: 14193535.3
(22) Date of filing: 17.11.2014
(51) Int. Cl.: A61B 6/00, G06T 7/00

(54) **Medical imaging system and program**

(30) Priority: 29.11.2013 JP 2013247273
(71) Applicant: Konica Minolta, Inc., Tokyo 100-7015 (JP)
(72) Inventor: Futamura, Hitoshi, Tokyo, Tokyo 100-7015 (JP); Tsunomori, Akinori, Tokyo, Tokyo 100-715 (JP)
(74) Representative: Alton, Andrew

(57) **Abstract**

An examination executor of an ultrasound examination is easily able to identify a position corresponding to an attention area on a medical image acquired in an X-ray examination on an imaging-object region, when the X-ray examination and the ultrasound examination are performed as separate examinations.

In the medical imaging system (100), the control part (31) of the server device (3), estimates a position of an attention area specified on a breast image in the image display device (4) in a breast as viewed from the front, on the basis of the position of the attention area in the breast image and an angle at which an X-ray photographing device is inclined during breast imaging. Then the control part (31) produces estimated clinical position information and causes the display part 43 of the image display device (4) to display the produced estimated clinical position information.

## Description

### Background of the Invention

### Field of the Invention

The present invention relates to a medical imaging system and a program.

### Description of the Related Art

Recently, a mammography examination and an ultrasound examination have mainly been performed for testing breast cancer. These examinations play a complementary role. It is expected that combined application of a mammography examination and an ultrasound examination in diagnosis will improve lesion detection accuracy.

Generally, in a combination examination, a mammography examination is firstly performed by a radiological technologist or the like, to obtain, on a radiographic image of a breast acquired by X-ray photography (hereinafter simply referred to as a "breast image"), objective positional information on an attention area such as an abnormal shadow candidate. After that, a medical technologist or the like performs an ultrasound examination on the breast, focusing on a position corresponding to the attention area on the breast image.

However, a mammography examination is usually performed on a compressed breast of a subject in a standing position. On the other hand, an ultrasound examination on a mammary gland is performed on a non-compressed breast of a subject in a supine position. Therefore, it is difficult, in an ultrasound examination, to locate a position corresponding to an attention area on a breast image acquired by a mammography examination.

To solve the above problem, JP 2011-200655 A, for example, discloses a medical imaging apparatus including an X-ray acquisition means and an ultrasound probe. The medical imaging apparatus determines a path between a position of the ultrasound probe and a position in a breast corresponding to an area of interest selected in a radiological image acquired by the X-ray acquisition means. Then the probe is guided along the determined path.

Also, JP 5015580 B2 discloses a method of producing a report image. The report image is produced by measuring spatial coordinates of an ultrasound probe, and combining an ultrasound image of a region to be diagnosed in an imaging-object tissue with a background image representing the imaging-object tissue, on the basis of information on the measured coordinates.

However, in a technique disclosed in JP 2011-200655 A, an ultrasound image is produced by placing an ultrasound probe on a breast which is in a state of being X-ray photographed in a mammography examination (i.e., an immobilized and compressed state). Therefore, when a lesion exists in a region closer to an X-ray image reception surface (panel), it is difficult to acquire a high quality ultrasound image, because it is impossible to irradiate a breast with ultrasound on the panel side. Moreover, as a breast is compressed at the time of X-ray photographing and remains in the same state throughout a series of tests from a mammography examination to an ultrasound examination, a patient suffers a great deal of discomfort. Consequently, the above technique is impracticable.

Moreover, in a technique disclosed in JP 5015580 B2, although it is possible to record coordinate information on a position in which an ultrasound image has been acquired, it is impossible to link the coordinate information with a position in a radiographic image, even for the same imaging-object region.

### Summary of the Invention

An object of the present invention is to facilitate, for an examination executor of an ultrasound examination, identifying a position in an imaging-object region corresponding to an attention area in a medical image acquired in an X-ray examination, when the X-ray examination and the ultrasound examination are performed separately on the same imaging-object region.

To achieve at least one of the abovementioned objects, according to an aspect, a medical imaging system reflecting one aspect of the present invention comprises: a display means configured to display at least one medical image acquired by X-ray photographing an imaging-object region; a specification means configured to specify an attention area in the medical image displayed by the display means; an estimation means configured to estimate a position of the attention area in the imaging-object region as viewed from the front, on the basis of a position of the attention area in the medical image and an inclination angle of an X-ray photographing device at the time of capturing the medical image; a production means configured to produce estimated clinical position information indicating the position of the attention area in the imaging-object region as viewed from the front, the position having been estimated by the estimation means; and a display control means configured to cause the display means to display the produced estimated clinical position information.

According to Item. 2, in the invention of Item. 1, the estimation means preferably estimates the position of the attention area in the imaging-object region as viewed from the front, by placing the medical image in an inclined manner with respect to a frontal image of the imaging-object region on the basis of the inclination angle of the X-ray photographing device, and by projecting the attention area in the medical image on the frontal image of the imaging-object region, and the production means preferably produces the estimated clinical position information on the basis of the frontal image of the imaging-object region on which the attention area has been projected.

According to Item. 3, in the invention of Item. 2, the frontal image of the imaging-object region is preferably divided into a plurality of segments, and the production means preferably calculates the size of an area occupied by the estimated position of the attention area in each of the segments, and produces information indicating which of the segments includes the area of the largest calculated size, the information being produced as the estimated clinical position information.

According to Item. 4, in the invention of any one of Items. 1 to 3, the imaging-object region is preferably a breast, and the medical image is preferably a breast image acquired by X-ray photographing the breast.

According to Item. 5, in the invention of Item. 4, the breast image preferably includes an MLO image captured by photographing a breast as the imaging-object region from an oblique direction, and a CC image captured by photographing the breast from a vertical direction, and the estimation means preferably places the MLO image in an inclined manner with respect to the frontal image of the imaging-object region on the basis of an inclination angle of the X-ray photographing device at the time of capturing the MLO image, as well as places the CC image on the basis of an angle of the X-ray photographing device at the time of capturing the CC image, projects the attention area in the MLO image and the attention area in the CC image on the frontal image of the imaging-object region, and estimates that an area in which projected images of the attention area overlap is a position of the attention area in the imaging-object region as viewed from the front.

According to Item. 6, in the invention of any one of Items. 1 to 5, the display control means preferably displays the estimated clinical position information on the same screen as the medical image on which the attention area is shown.

According to Item. 7, in the invention of any one of Items. 1 to 6, the display means preferably displays an area of the abnormal shadow candidate detected by the detection means on the medical image.

According to Item. 8, in the invention of any one of Items. 1 to 6, the medical imaging system preferably further comprises an input means configured to input an area of an abnormal shadow candidate through the medical image, and the display means preferably displays the area of the abnormal shadow candidate input by the input means on the medical image.

To achieve at least one of the abovementioned objects, according to an aspect, a program reflecting one aspect of the present invention causes a computer to function as: a display means configured to display at least one medical image acquired by X-ray photographing an imaging-object region; a specification means configured to specify an attention area in the medical image displayed by the display means; an estimation means configured to estimate a position of the attention area in the imaging-object region as viewed from the front, on the basis of a position of the attention area in the medical image and an inclination angle of an X-ray photographing device at the time of capturing the medical image; a production means configured to produce estimated clinical position information indicating the position of the attention area in the imaging-object region as viewed from the front, the position having been estimated by the estimation means; and a display control means configured to cause the display means to display the produced estimated clinical position information.

### Brief Description of the Drawings

The above and other objects, advantages and features of the present invention will become more fully understood from the detailed description given hereinbelow and the appended drawings which are given by way of illustration only, and thus are not intended as a definition of the limits of the present invention, and wherein:
Fig. 1 is a diagram showing an example of the entire configuration of a medical imaging system in an embodiment of the present invention;
Fig. 2 is a block diagram showing a function configuration of a server device in Fig. 1;
Fig. 3 is a block diagram showing a function configuration of an image display device in Fig. 1;
Fig. 4A is an image formed of a right-breast MLO image and a left-breast MLO image arranged side by side and joined along the chest wall sides, and Fig. 4B is an image formed of a right-breast CC image and a left-breast CC image arranged side by side and joined along the chest wall sides;
Fig. 5 is a flowchart showing a flow of processing for producing estimated clinical position information, executed by a control part in Fig. 2;
Fig. 6 is a figure showing an example of a viewer screen which appears on the image display device in Fig. 1;
Fig. 7 is a flowchart showing a flow of processing executed in step S6 in Fig. 5;
Fig. 8A is a drawing for explaining coordinates in a breast image, and Fig. 8B is a drawing for explaining detecting a position of a nipple;
Fig. 9 is a drawing for explaining a method of producing clinical position information on an attention area;
Fig. 10A is a drawing showing a frontal schema image of a right breast, and Fig. 10B is a drawing showing a frontal schema image of a left breast;
Fig. 11 is a drawing for explaining a method of excluding an area having a low number of abnormal shadow candidates from a clinical position corresponding to an attention area;
Figs. 12A, 12B and 12C are drawings showing examples of clinical position information; and
Figs. 13A and 13B are figures showing examples of a viewer screen showing clinical position information on an attention area.

### Description of the Preferred Embodiments

Hereinafter, an embodiment of the present invention will be described with reference to the drawings. However, the scope of the invention is not limited to the illustrated examples.

### (Configuration of medical imaging system 100)

First, a configuration of an embodiment of the present invention is described below.

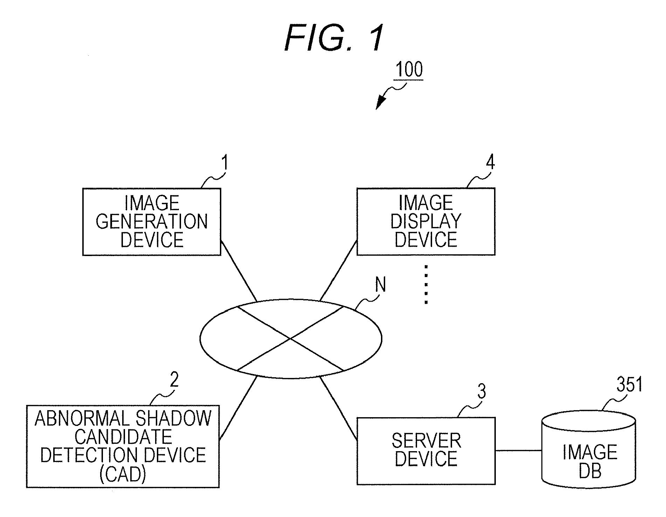

Fig. 1 shows a system configuration of a medical imaging system 100 in the embodiment.

As shown in Fig. 1, the medical imaging system 100 includes an image production device 1, an abnormal shadow candidate detection device 2, a server device 3, and an image display device 4. The above devices 1 to 4 are interconnected via a communication network N such as a local area network (LAN) constructed in a medical facility, so as to enable mutual transmission and reception of data. For the communication network N, standards of Digital Imaging and Communication in Medicine (DICOM) are applied. In the medical facility, an ultrasonic diagnostic device 5 (not shown) is provided. The number of each device is not specifically limited.

The medical imaging system 100 is configured to X-ray photograph an imaging-object region, and store and manage an acquired medical image. The system also produces estimated clinical position information by estimating a clinical position of an attention area such as an abnormal shadow candidate on the medical image. Then the system displays the estimated clinical position information to provide assistance in an examination performed with the ultrasonic diagnostic device 5 on a subject in a supine position. The clinical position means positional information on an imaging-object region in an anterior view of a human body. While the medical imaging system 100 is able to manage medical images of various imaging-object regions, a description is provided, as an example in the embodiment, mainly on X-ray photographing a breast for assisting in an ultrasound examination on the breast on the basis of an acquired breast image.

The following is a description of the devices 1 to 4 included in the medical imaging system 100.

The image production device 1 is an X-ray photographing device to X-ray photograph an imaging-object region in a human body, and to produce digital data of a captured image (medical image). A modality such as a computed radiography (CR) device or a flat panel detector (FPD) device can be used as the image production device 1. In the embodiment, a FPD device is applied as the image production device 1. The FPD device performs X-ray photographing of right and left breasts, generating data of a breast image as a medical image.

The image production device 1 is in conformity to the aforementioned DICOM standards. It is possible for the device to externally input or automatically produce various kinds of accompanying information, such as patient data and examination data, to be appended to produced medical images. Patient data includes patient identification data (patient ID, for example) for identifying a patient (subject), and other data such as name, sex, and date of birth of the patient. Examination data includes examination identification data (examination ID, for example) for identifying an examination, and other data such as date of examination, examination condition (examined region, laterality (right/left), direction (e.g., vertical direction (CC), and oblique direction (MLO)), photographing angle, and modality type. In the embodiment, a set of images (right and left MLO images, and right and left CC images) acquired in a mammography examination on the same patient is managed with the same examination ID. The image production device 1 appends information such as the patient data, the examination data, and a unique ID (UID) for identifying an image to a produced medical image as header information. Then the image production device 1 transmits the medical image with the header information, via the communication network N, to the abnormal shadow candidate detection device 2 and the server device 3. When the image production device 1 does not conform to DICOM standards, it is possible to input the above accompanying information to the image production device 1 by using a DICOM converter which is not shown.

The abnormal shadow candidate detection device (CAD) 2 as a detection means is a computer which performs image analysis on a medical image provided by the image production device 1 and executes processing for detecting an abnormal shadow candidate. The abnormal shadow candidate detection device 2 includes a storage part such as a central processing unit (CPU), a random access memory (RAM), and a hard disk drive (HDD), and a communication part such as a LAN card. The storage part of the abnormal shadow candidate detection device 2 stores a detection program with a detection algorithm appropriate for each type of an abnormal shadow. The CPU of the abnormal shadow candidate detection device 2 executes processing for detecting an abnormal shadow candidate by cooperating with the detection program stored in the storage part, to detect an abnormal shadow candidate in each medical image which is input to the device through the communication part. For example, the abnormal shadow candidate detection device 2 detects an abnormal shadow candidate of a tumor and a microcalcification cluster in a breast image.

A publicly known algorithm is applicable for detecting an abnormal shadow candidate. For example, as an algorithm for detecting a tumor shadow candidate in a breast image, a method using an iris filter, disclosed in JP 10-91758 A, a method using a Laplacian filter (The Institute of Electronics, Information and Communication Engineers (IEICE) Transactions (D-II), Vol. J76-D-II, no. 2, pp. 241-249, 1993), and the like are applicable. As a detection algorithm for detecting a microcalcification cluster shadow candidate, methods using a morphology filter (IEICE Transactions (D-II), Vol. J75-D-II, no. 7, pp. 1170-1176, 1992), a Laplacian filter (IEICE Transactions (D-II), Vol. J71-D, no. 10, pp. 1994-2001, 1988), a triple ring filter, and the like are applicable.

When detection of an abnormal shadow candidate is completed, the CPU of the abnormal shadow candidate detection device 2 produces abnormal shadow candidate information on the basis of a result of the detection of an abnormal shadow candidate. The abnormal shadow candidate information includes, for example, positional information of an area of a detected abnormal shadow candidate, and information on the type of the abnormal shadow candidate (tumor, microcalcification cluster, and the like). Then, the abnormal shadow candidate detection device 2 transmits the produced abnormal shadow candidate information to the server device 3 through the communication part. At this time, the abnormal shadow candidate detection device 2 appends the header information (UID, at least) on a medical image in which the abnormal shadow candidate has been detected to the abnormal shadow candidate information.

The server device 3, with the image display device 4 which is a client, configures a picture archiving and communication system (PACS). The server device 3 links a medical image produced in the image production device 1 with the accompanying information and the abnormal shadow candidate information. Then the server device 3 stores the medical image in a database (an image DB 351), and manages such medical image. The server device 3 also causes the image display device 4 to display a selection screen for selecting a medical image related to an examination to be handled, or a viewer screen for displaying a medical image related to a selected examination. The server device 3 subsequently executes various kinds of processing including processing for producing estimated clinical position information which will be described later, depending on operation information transmitted from the image display device 4.

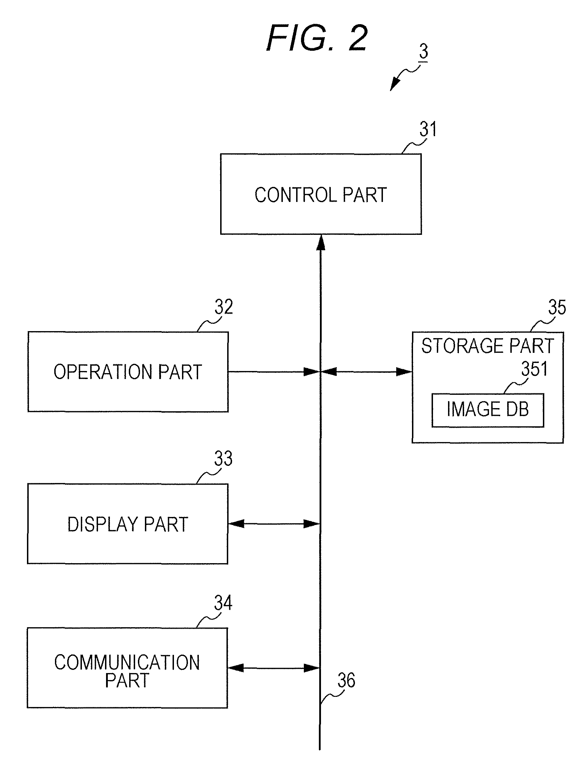

Fig. 2 shows an example of a function configuration of the server device 3. As shown in Fig. 2, the server device 3 includes a control part 31, an operation part 32, a display part 33, a communication part 34, and a storage part 35. The above parts are connected with one another by a bus 36.

The control part 31 includes a central processing unit (CPU), a random access memory (RAM), and the like. The CPU of the control part 31 reads various kinds of programs such as a system program and a processing program stored in the storage part 35, decompresses such programs in the RAM, and executes various kinds of processing by following the decompressed programs. The control part 31 functions as an estimation means, a production means, and a display control means, by executing the processing for producing estimated clinical position information which will be described later.

The operation part 32 includes a keyboard provided with character input keys, numeral input keys, various function keys, and the like, and a pointing device such as a mouse. The operation part 32 outputs a depression signal from a key pressed on the keyboard or an operation signal from the mouse, as an input signal, to the control part 31.

The display part 33 includes a monitor such as a cathode ray tube (CRT) display or a liquid crystal display (LCD). The display part 33 displays various kinds of screens, following an instruction of a display signal which is input from the control part 31.

The communication part 34 includes a LAN card and the like. The communication part 34 transmits/receives data to/from an external device connected with the communication network N via a switching hub.

The storage part 35 includes a hard disk drive (HDD), a semiconductor non-volatile memory, and the like. As previously mentioned, the storage part 35 stores various kinds of programs. The storage part 35 also includes the image DB 351.

The image DB 351 is a database to store a medical image. For example, the image DB 351 has an image management table to store management information regarding individual medical images stored in the image DB 351. In the image management table, management information on each medical image is stored as one record. The management information includes an UID, patient data, examination data, and file data (e.g., name of a file containing a medical image, name of a file containing abnormal shadow candidate information corresponding to the medical image, file storage place, update date, and file size).

When a medical image from the image production device 1 is received through the communication part 34, the control part 31 stores the received medical image in the image DB 351, produces management information on the basis of header information of the received medical image, and stores the management information in the image management table in the image DB 351. Also, when abnormal shadow candidate information from the abnormal shadow candidate detection device 2 is received through the communication part 34, the control part 31 stores the received abnormal shadow candidate information in the image DB 351, retrieves, from the image management table, a record having an UID corresponding to an UID contained in the abnormal shadow candidate information, and writes the name of a file containing the abnormal shadow candidate information, a storage place of the file, and the like, to the record which has been retrieved. As a result, the image DB 351 stores a medical image and abnormal shadow candidate information on an abnormal shadow candidate detected in the medical image, in a linked and retrievable manner.

The image display device 4 displays a selection screen, a viewer screen, and the like transmitted from the server device 3. The image display device 4 also transmits, to the server device 3, operation information which is input through the above screens. As the server device 3 sends back a result of processing executed in response to an operation, the image display device 4 displays the result on the viewer screen. The image display device 4 functions as a display means and a specification means.

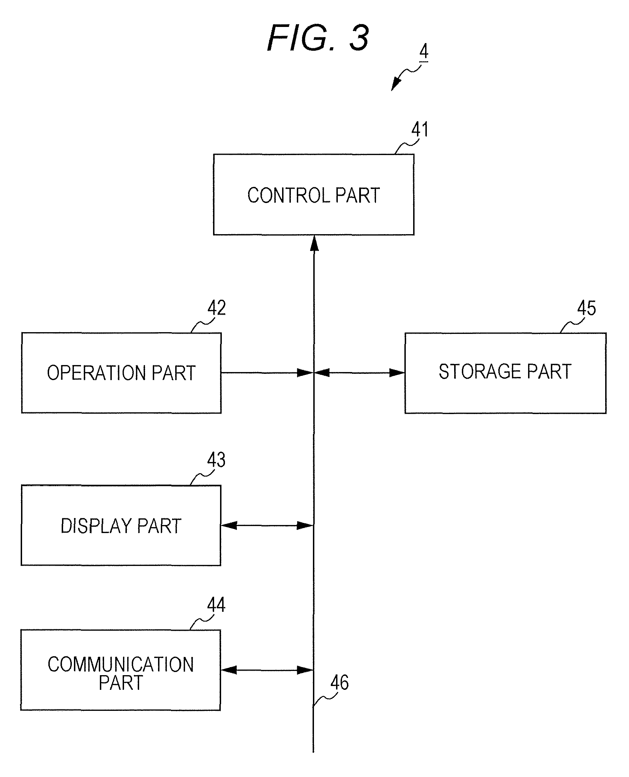

Fig. 3 shows an example of a function configuration of the image display device 4. As shown in Fig. 3, the image display device 4 includes a control part 41, an operation part 42, a display part 43, a communication part 44, and a storage part 45. The above parts are connected with one another by a bus 46.

The control part 41 includes a CPU, a RAM, and the like. The CPU of the control part 41 reads various kinds of programs such as a system program and a processing program stored in the storage part 45, decompresses such programs in the RAM, and executes various kinds of processing by following the decompressed programs.

The operation part 42 includes a keyboard provided with character input keys, numeral input keys, various function keys, and the like, and a pointing device such as a mouse. The operation part 42 outputs a depression signal from a key pressed on the keyboard or an operation signal from the mouse, as an input signal, to the control part 41.

The display part 43 includes a monitor such as a cathode ray tube (CRT) display or a liquid crystal display (LCD). The display part 43 displays various kinds of screens, following an instruction of a display signal which is input from the control part 41.

The communication part 44 includes a LAN card and the like. The communication part 44 transmits/receives data to/from an external device connected with the communication network N via a switching hub.

The storage part 45 includes a hard disk drive (HDD), a semiconductor non-volatile memory, and the like. As previously mentioned, the storage part 45 stores a system program and other various kinds of programs (for example, a program to display various kinds of screens received from the server device 3, and a program to transmit operation information which has been input from the operation part 42 to the server device 3 through the communication part 44).

(Operation of the medical imaging system 100)

The following is a description of operation of the medical imaging system 100.

In the meantime, in a breast cancer test, there is usually conducted a combination examination in which two examinations, i.e., a mammography examination and an ultrasound examination, are conducted in combination. In such combination examination, generally, a radiological technologist performs a mammography examination first, to obtain objective positional information of an attention area such as an abnormal shadow candidate, in an acquired breast image. After that, a medical technologist observes the breast image, then performs an ultrasound examination on a breast, focusing on a position in the breast, which corresponds to the attention area on the breast image. When both of the two examinations have finished, a doctor interprets the breast image and an ultrasound image of the abnormal shadow candidate, and makes a final diagnosis.

In a mammography examination, in general, photographing is performed by the image production device 1 four times in total for one patient. Specifically, each of right and left breasts is photographed in both an oblique direction (MLO) and a vertical direction (CC), to produce two breast images for each of the right and left breasts. At the time of photographing, the patient is in a standing position, with a breast compressed. On the other hand, an ultrasound examination is performed on a patient in a supine position with non-compressed breasts. Therefore, a medical technologist or the like, as an examination executor who conducts an ultrasound examination, has to perform the examination by intuitively converting a position of an attention area on the breast image into a clinical position which is a position in the breast in an anterior view of a human body.

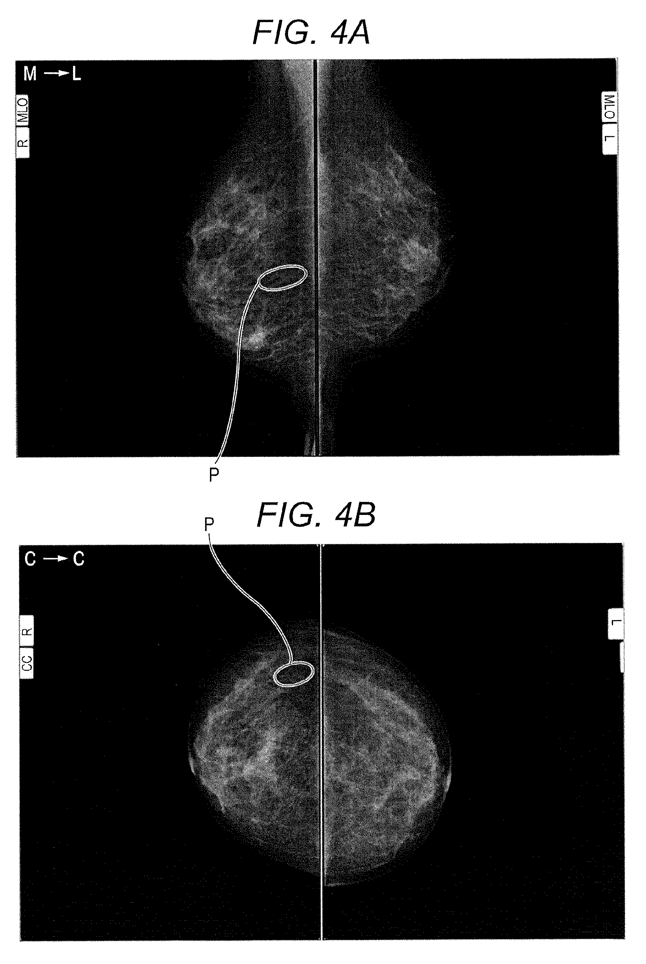

Fig. 4A shows an image in which right and left MLO images are arranged side by side and joined along the chest wall. Fig. 4B shows an image in which right and left CC images are arranged side by side and joined along the chest wall. As shown in Figs. 4A and 4B, when a breast image is observed, the image is placed for observation normally in such a manner that each edge thereof is parallel to a corresponding edge of the display part (monitor).

In the image production device 1, a CC image is acquired by irradiating a breast from directly above, when a subject is in a standing position. In Fig. 4B, right and left CC images are arranged side by side so as to be joined along the chest wall. Also, the images are arranged such that each edge thereof is parallel to a corresponding edge of the monitor. In addition, positions of right and left breasts appearing in the CC images correspond to those in the MLO images (right and left breast images are on the left and the right, respectively, as seen from the front). Accordingly, the upper side and the lower side of the CC images correspond to the lateral side and the medial side of the breasts, respectively. Therefore, an observer is able to estimate, on the basis of the CC images, whether an attention area is in the lateral side or the medial side of a breast. For example, in the CC images shown in Fig. 4B, it can be seen that an attention area P which should be focused on exists in the lateral side of the right breast.

On the other hand, an MLO image is acquired by photographing a breast from an oblique direction at an optimum photographing angle at each photographing time. Here, assuming that an MLO image is captured by photographing a breast from a straight lateral direction (in other words, the direction of X-rays in MLO imaging is orthogonal to that in CC imaging), it is possible, in Fig. 4A, to estimate a position of the attention area P in a vertical direction (head-foot direction) in the breast, on the basis of the MLO images placed such that each edge thereof is parallel to a corresponding edge of the monitor. Accordingly, in this case, it is possible to locate an approximate position in which the attention area P exists, by observing the MLO images and the CC images together. However, as previously mentioned, an MLO image is acquired by photographing a breast as an imaging object from an oblique direction. Therefore, actually, it is difficult to estimate a vertical position in the breast by observing an MLO image placed as shown in Fig. 4A. Information on a photographing angle is contained in accompanying information for a breast image. However, even if the photographing angle has been obtained, it takes time to estimate a position of an attention area P on the basis of the photographing angle. This results in lowered efficiency of the examination. Moreover, the position is highly likely to be estimated erroneously.

If an ultrasound examination is performed by applying a probe on a wrong position, no shadow corresponding to an attention area will be observed. This could lead to various disadvantages such as making a patient nervous, making an examination executor confused, a prolonged examination, and an incomplete examination.

In view of the foregoing, according to the embodiment, a clinical position of an attention area is estimated on the basis of a breast image acquired in a mammography examination to obtain estimated clinical position information, and the estimated clinical position information is displayed, so that it becomes easy for an examination executor to identify a clinical position corresponding to the attention area in the breast.

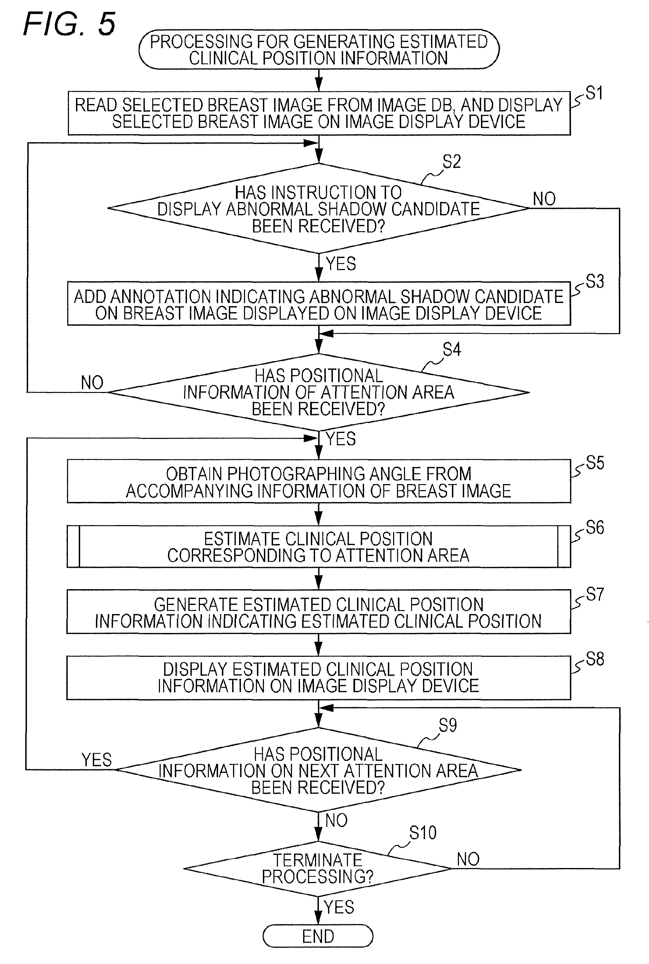

Fig. 5 is a flowchart showing a flow of processing for producing estimated clinical position information, executed by the server device 3. The processing for producing estimated clinical position information is executed in cooperation between the control part 31 and a program stored in the storage part 35, when a breast image related to an examination to be handled is selected through the image display device 4 and information (such as examination ID) for specifying the selected breast image related to the examination is received through the communication part 34.

First, the control part 31 retrieves and reads the selected breast image (right and left MLO images and CC images) from the image DB 351, produces screen information on a viewer screen 431 on which the read breast image has appeared, transmits the screen information to the image display device 4 which is the requestor through the communication part 34, and causes the display part 43 of the image display device 4 to display the viewer screen 431 on which the selected breast image appears (step S1). Meanwhile, the control part 31 of the server device 3 temporarily stores in a memory the examination ID and the UID of the breast image displayed on the image display device 4, until an instruction to exit is received from the image display device 4.

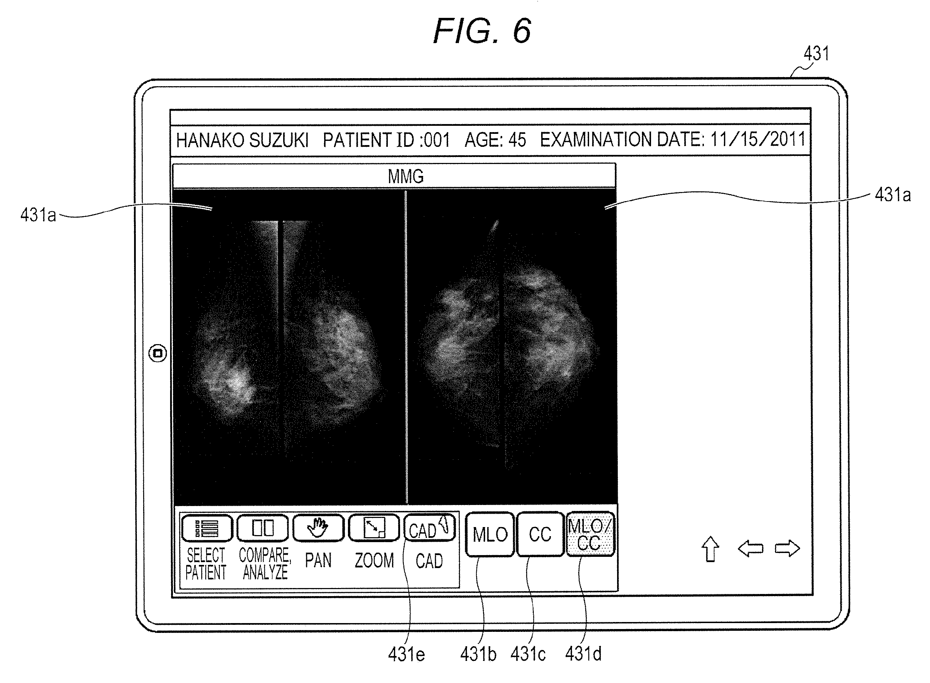

Fig. 6 shows an example of the viewer screen 431 to be displayed on the image display device 4. In the viewer screen 431, there are provided an image display field 431a, an MLO button 431b for instructing to display an MLO image only, a CC button 431c for instructing to display a CC image only, an MLO/CC button 431d for instructing to display both an MLO image and a CC image, a CAD button 431e for instructing to indicate an abnormal shadow candidate. An examination executor (operator) such as a medical technologist is able to input an instruction to indicate an abnormal shadow candidate by pressing a CAD button 431e, for example, through the operation part 42. When the instruction is input, an abnormal shadow candidate is indicated on a breast image displayed on the screen, so that the examination executor is able to check the abnormal shadow candidate shown on the breast image.

When the control part 31 receives from the image display device 4, through the communication part 34, operation information indicating an instruction to show an abnormal shadow candidate (Yes in step S2), the control part 31 retrieves and reads, from the image DB 351, an abnormal shadow candidate information corresponding to the breast image displayed on the image display device 4. The control part 31 then produces screen information for displaying a screen on which an annotation indicating a position of the abnormal shadow candidate appears on the breast image displayed on the viewer screen 431. Then, the control part 31 transmits the screen information, through the communication part 34, to the image display device 4 which is the requestor, and causes the display part 43 of the image display device 4 to display the viewer screen 431 on which an annotation indicating the position of the abnormal shadow candidate appears on the breast image (step S3).

Subsequently, the control part 31 determines whether or not positional information on an attention area has been received from the image display device 4 through the communication part 34 (step S4). In the image display device 4, when an attention area is specified, through the operation part 42, on the breast image displayed on the viewer screen 431, positional information on the specified attention area is transmitted as operation information to the server device 3 through communication part 44. An attention area means an area which should be focused on in an ultrasound examination, such as an area of an abnormal shadow candidate. Examination executor specifies, through the operation part 42, two areas as attention areas when an identical abnormal shadow candidate is confirmed in each of MLO and CC images, while specifying a single area as an attention area when an abnormal shadow candidate is confirmed in only one of the MLO and CC images.

When the control part 31 determines that the positional information on an attention area has been received from the image display device 4 through the communication part 34 (Yes in step S4), the control part 31 obtains a photographing angle contained in accompanying information of each breast image (right or left breast images in which the attention area has been specified) displayed on the image display device 4 (step S5). Then the control part 31 estimates clinical position information which corresponds to the attention area on the breast images, on the basis of the positional information of the attention area and the photographing angles of the breast images (step S6). Photographing angle means an angle at which the image production device 1 is inclined at the time of photographing a breast (an inclination of a direction of X-rays). In the image production device 1 in its initial state, a radiation source which emits X-rays, an object table for supporting an imaging object, and a FPD device for detecting X-rays which have passed through the imaging object are arranged in a vertical row in downward order. When taking an MLO image, the above components are integrally inclined at an angle according to an angle at which a breast as an imaging object is photographed. When taking a CC image, the image production device 1 is not inclined and remains in its initial state. In other words, a photographing angle is zero degree (0°).

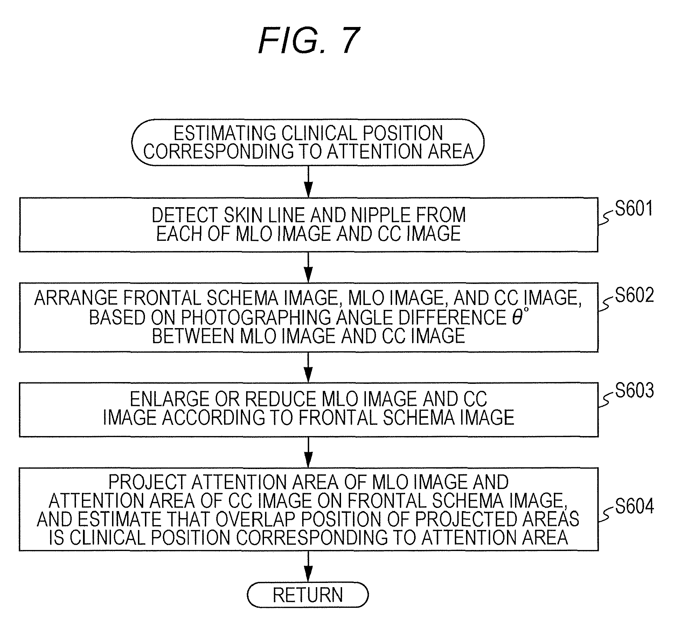

An estimation of a clinical position corresponding to an attention area is conducted in step S6 by executing processing shown in Fig. 7. Fig. 7 shows a flowchart of the processing for estimating a clinical position in step S6.

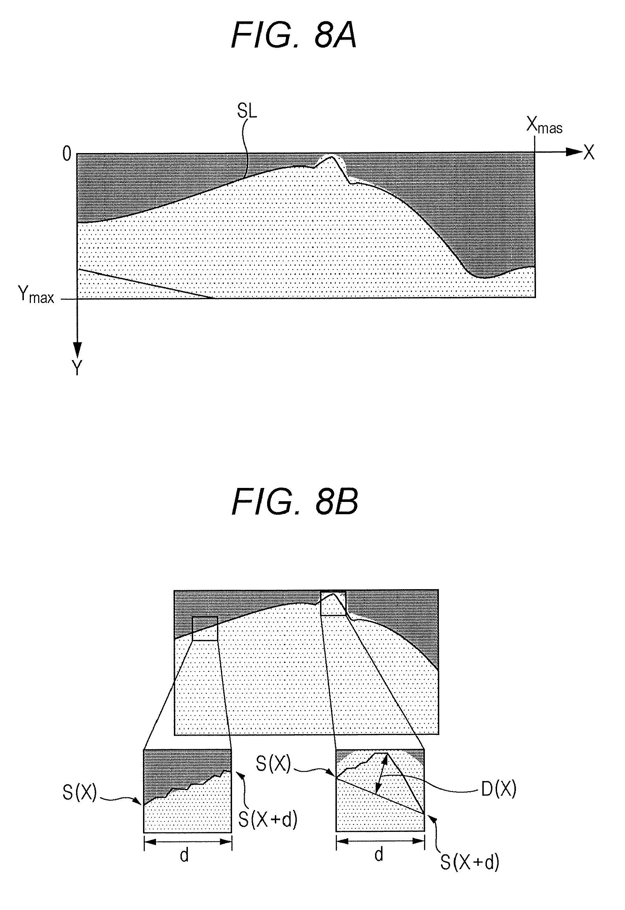

First, the control part 31 detects a skin line and nipple from a breast image (an MLO image and a CC image) (step S601). As shown in Fig. 8A, a position of each pixel in the breast image is represented in coordinates (X, Y) with the X-axis in a vertical direction of a breast in the breast image and the Y-axis in a direction perpendicular to the X-axis. A pixel value of coordinates (X, Y) is represented as V(X, Y). Further, a coordinate of an end of the image in each of the X-axis and Y-axis directions is represented as Xmax and Ymax, respectively.

In step S601, first, the control part 31 performs, in the breast image, filter processing with a Sobel filter for each pixel as a focused pixel. Next, the control part 31 searches the Sobel-filtered breast image, at each X-coordinate (0 to Xmax) in the Y direction, for coordinates (S) with the largest pixel value V(X, Y). Then the control part 31 extracts the coordinates (S) with the largest pixel value V(X, Y) as an edge at each X-coordinate. The extracted edges form a skin line SL which is a boundary between a breast area and an out-of-breast area. Subsequently, as shown in Fig. 8B, the control part 31 calculates, for each point S (X) on the skin line SL, distances D between a straight line connecting S(X) and S(X+d (d may be 10, for example)) and each point between S(X) and S (X+d) (i.e., S(X+1), S(X+2) ... S(X+d-1)). Then the control part 31 detects a position of S(X) for which the largest distance D(X) of the calculated distances D has the greatest value among all the points S (X). The control part 31 determines that the detected position S(X) is a position of the nipple.

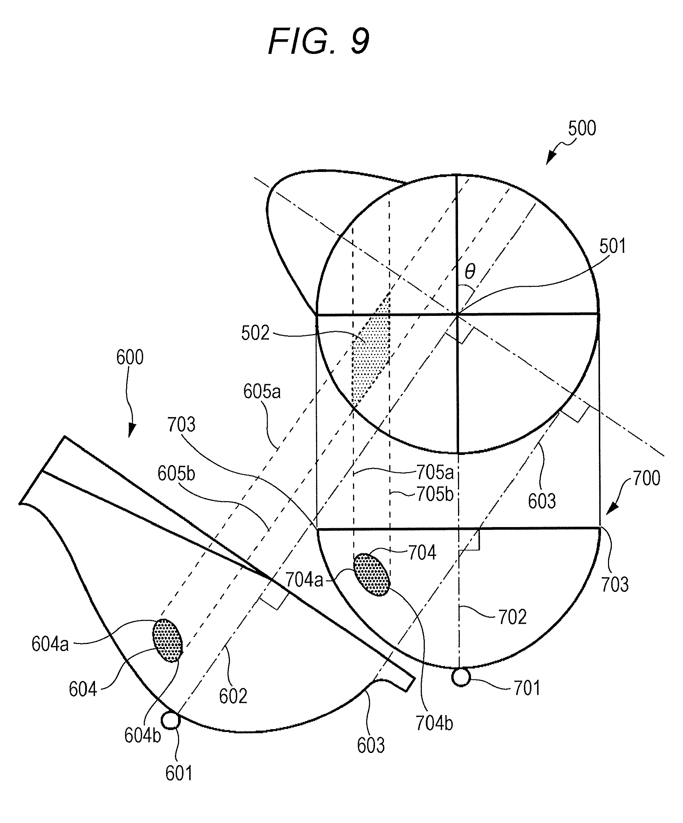

Subsequently, as shown in Fig. 9, the control part 31 arranges a frontal schema image (500 in Fig. 9), an MLO image (600 in Fig. 9), and a CC image (700 in Fig. 9) of the breast on the RAM, on the basis of an angle difference θ [°] between a photographing angle at the time of taking an MLO image and a photographing angle at the time of taking a CC image (step S602). The angle difference θ [°] corresponds to a photographing angle at the time of taking an MLO image.

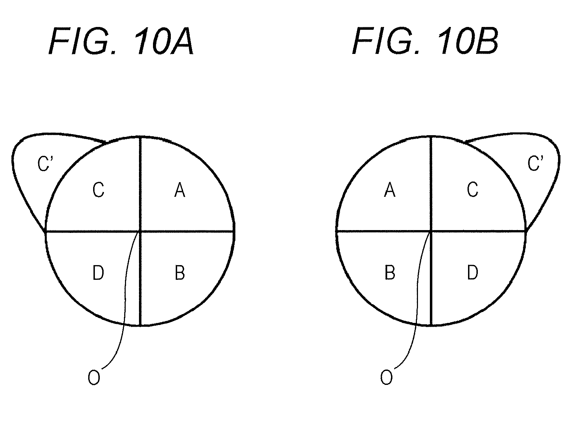

Figs. 10A and 10B are examples of frontal schema images of right and left breasts, respectively. Each of the frontal schema images of right and left breasts schematically shows each of the breasts in a frontal view, on which a clinical position of an attention area is superimposed. In the frontal schema image, a circle represents a breast, and the center of the circle represents a position of a nipple. Areas A, B, C, and D represent an upper medial area, a lower medial area, an upper lateral area, and a lower lateral area of a breast, respectively. An area C' adjacent to the area C represents an armpit. The frontal schema image of a breast has a configuration similar to a body mark for indicating a position in which an ultrasound probe is placed, that is, an inspection position, in an ultrasound image acquired in an ultrasound diagnosis. Therefore, the frontal schema image makes it easy for a medical technologist who performs an ultrasound examination to locate a position in a breast.

As shown in Fig. 9, in step S602, firstly, a frontal schema image 500 is placed in the RAM. Next, an MLO image 600 and a CC image 700 are arranged, in such a manner that both of a perpendicular line 602 drawn from a nipple 601 to a chest wall in the MLO image 600 and a perpendicular line 702 drawn from a nipple 701 to a chest wall in the CC image 700 extend to pass through a nipple 501 in the frontal schema image 500, and, at the same time, an angle difference between the above perpendicular lines is equal to an angle difference θ [°] between a photographing angle at the time of taking an MLO image and a photographing angle at the time of taking a CC image. In other words, each of the MLO image 600 and the CC image 700 is placed so as to be inclined at the corresponding photographing angle with respect to the frontal schema image 500.

Next, the control part 31 enlarges or reduces the MLO image 600 and the CC image 700 in size according to a size of the frontal schema image 500 (step S603). Specifically, the CC image 700 is enlarged or reduced in such a manner that both ends 703 of a breast area in the CC image 700 align with both ends of the circle of the frontal schema image 500, while the perpendicular line 702 drawn from the nipple 701 to the chest wall extends to pass through the nipple 501 in the frontal schema image 500. The MLO image 600 is enlarged or reduced in such a manner that a perpendicular line 603 drawn from a lower end of a breast area to the chest wall in the MLO image 600 extends to be tangent to the circle of the frontal schema image 500, while the perpendicular line 602 drawn from the nipple 601 to the chest wall extends to pass through the nipple 501 in the frontal schema image 500. Meanwhile, the both ends 703 of the breast area in the CC image 700 and the lower end of the breast area in the MLO image 600 are specified on the basis of the detected skin line.

Next, the control part 31 projects the attention area 604 of the MLO image 600 and the attention area 704 of the CC image 700 on the frontal schema image 500, and estimates that a position in which the projected attention areas overlap is a clinical position corresponding to the attention areas (step S604). Specifically, first, in the MLO image 600, straight lines 605a and 605b which are perpendicular to the chest wall are drawn from a cranial end 604a and a caudal end 604b of the attention area 604, respectively, so that a strip-shaped area bordered by the straight lines 605a and 605b is projected on the frontal schema image 500. Likewise, in the CC image 700, straight lines 705a and 705b which are perpendicular to the chest wall are drawn from a lateral end 704a and a medial end 704b of the attention area 704, respectively, so that a strip-shaped area bordered by the straight lines 705a and 705b is projected on the frontal schema image 500. Then, an area 502 in which the projected strip-shaped areas overlap is estimated to be a clinical position corresponding to the attention areas. When an attention area is specified in only one of the MLO image and the CC image, a corresponding one of the abovementioned strip-shaped areas is estimated to be a clinical position corresponding to the attention area.

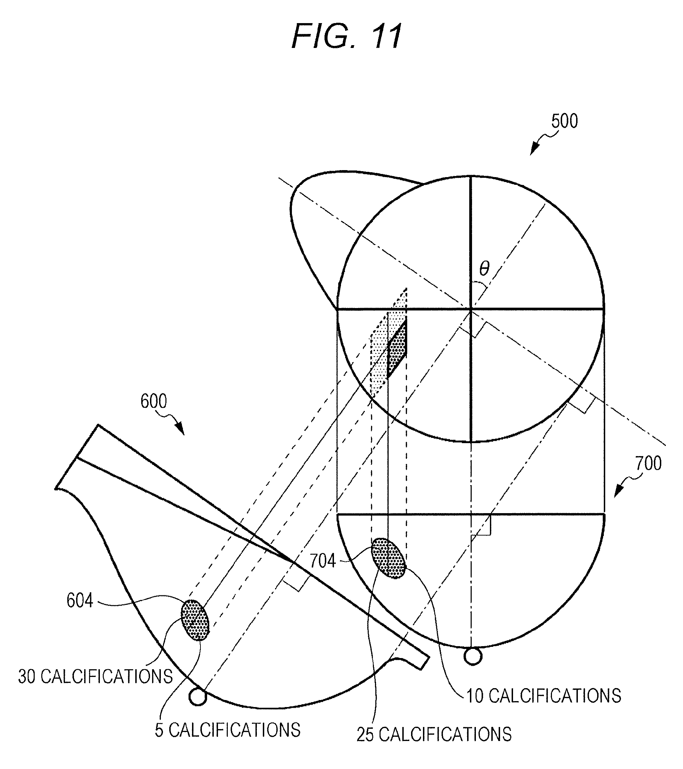

Additionally, as shown in Fig. 11, the attention area 604 and the attention area 704 may be divided into separate sections, and projected on the frontal schema image 500. Then, the number of shadows included in each section (the number of calcification, for example) is counted. A section having the smallest number of shadows overlapping in the frontal schema image 500 may be regarded as a section having the lowest probability of existence of an abnormal shadow candidate, and may be excluded from the position corresponding to the attention area.

In Fig. 9, the attention area 604 in the MLO image 600 is located above the nipple 601 in the MLO image 600, and the attention area 704 in the CC image 700 is located laterally outward from the nipple 701 in the CC image 700. Therefore, an observer is apt to think intuitively that the attention area is located in the area C. However, taking the photographing angles into consideration, the clinical position corresponding to the attention area is more likely to exist actually in the area D. Consequently, by conducting the processing in step S6, it is possible, taking account of photographing angles, to locate a clinical position in which an attention area is more likely to really exist, when it is difficult to locate a clinical position corresponding to the attention area by only depending on human intuition.

When has finished estimating an estimated clinical position corresponding to an attention area, the control part 31 moves to step S7 in Fig. 5, to produce estimated clinical position information which indicates an estimated clinical position (step S7).

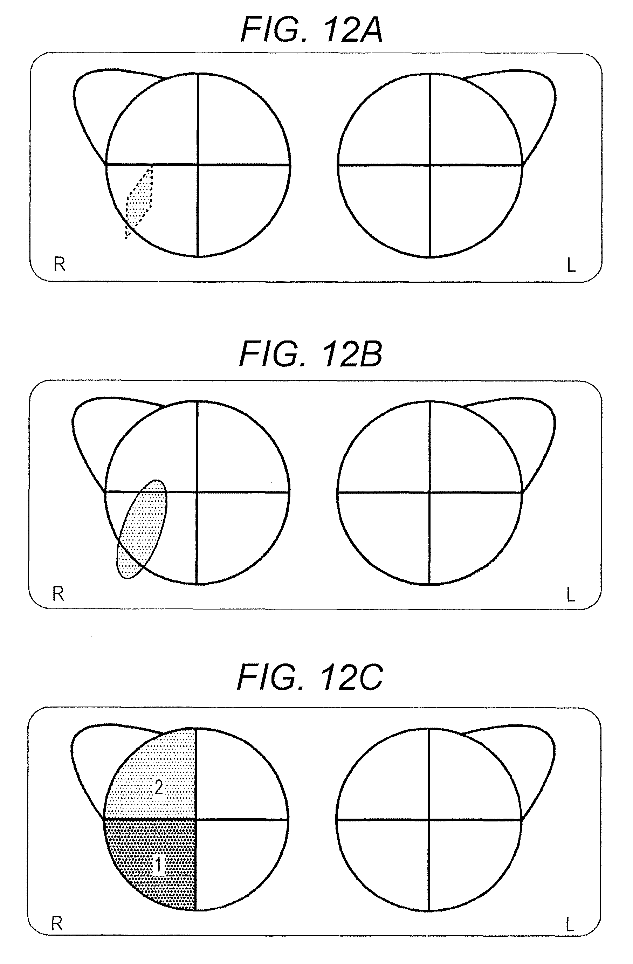

Figs. 12A, 12B and 12C are drawings showing examples of estimated clinical position information. As shown in Fig. 12A, the estimated clinical position information may be indicated by enclosing an estimated clinical position (parallelogram area) in the frontal schema image, or overlaying the estimated clinical position with color or the like. As shown in Fig. 12B, the estimated clinical position information may be produced by drawing an ellipse which covers the area of the estimated clinical position and has the major axis (the longer axis) inclining at the same angle as the longer diagonal of the parallelogram indicating the clinical position, such that the ellipse overlaps the frontal schema image. As shown in Fig. 12C, the estimated clinical position information may be produced by calculating the size (or counting the number of pixels) of the area occupied by the clinical position in each area A to D in the frontal schema image, to obtain information indicating one of the areas A to D in which the calculated size is largest (or alternatively, information indicating the order of the calculated size) as the estimated clinical position information.

When has finished producing the estimated clinical position information, the control part 31 produces screen information on the viewer screen 431 on which the produced estimated clinical position information is displayed, transmits the screen information to the image display device 4 through the communication part 34, and causes the estimated clinical position information to appear on the viewer screen 431 of the display part 43 (step S8).

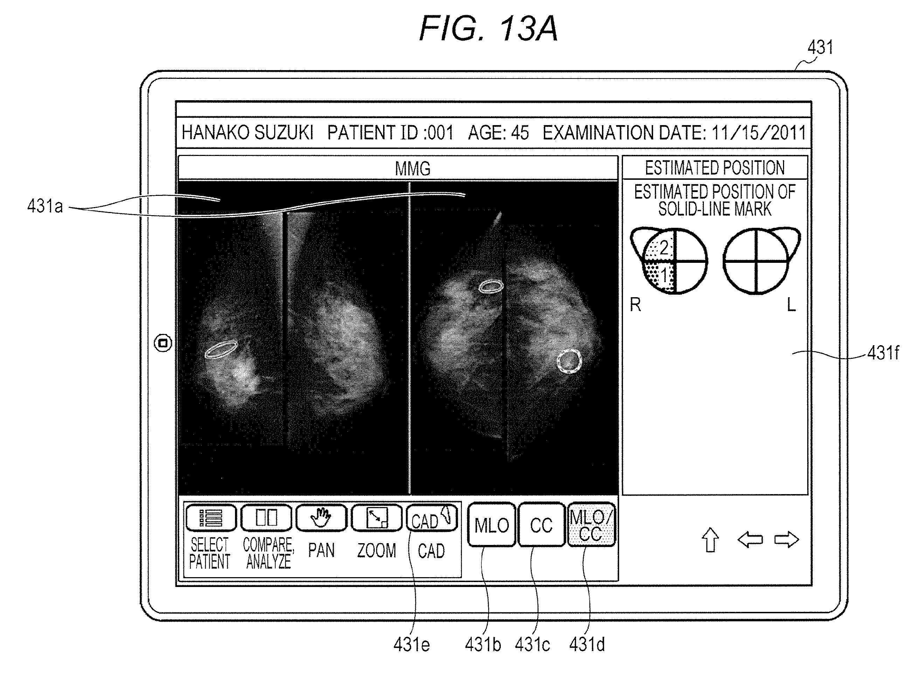

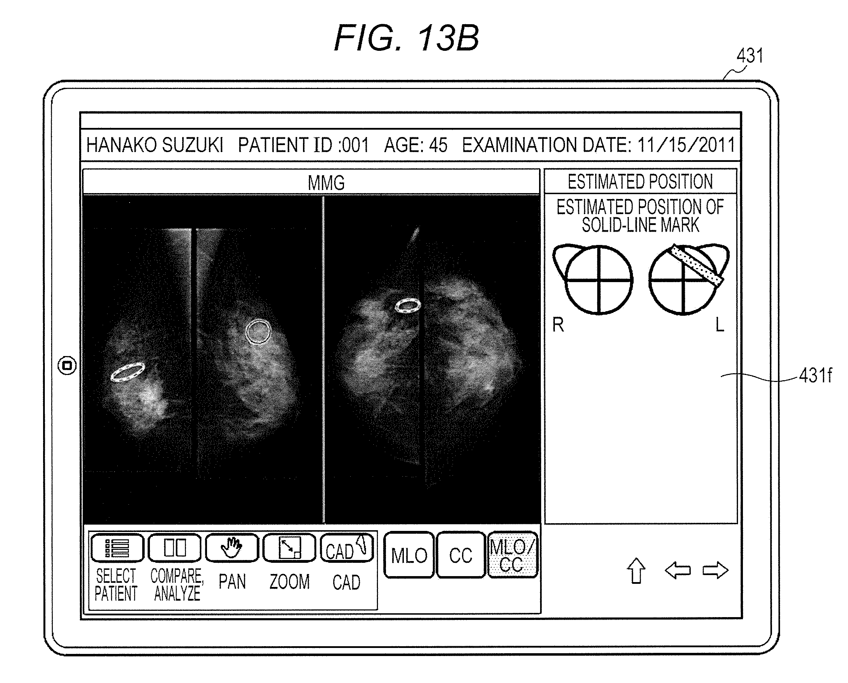

Figs. 13A and 13B are figures showing examples of the viewer screen 431 displaying the estimated clinical position information. Fig. 13A shows, in a field 431f on the viewer screen 431, an example of the estimated clinical position information produced on the basis of an attention area (shown by solid lines) specified by two kinds of breast images, i.e., an MLO image and a CC image. Fig. 13B shows an example of the estimated clinical position information produced on the basis of an attention area (shown by a solid line) specified by an MLO image only.

As a result of displaying the estimated clinical position information indicating a clinical position of an attention area, as shown in Figs. 13A and 13B, an examination executor is able to easily know about where in a breast a specified attention area is located. This prevents performing an ultrasound examination in a wrong position in the breast, and increases examination efficiency. Furthermore, as a result of displaying a breast image on which an annotation indicating a specified attention area appears and estimated clinical position information in such a manner that the breast image and the estimated clinical position information are arranged side by side on the same screen, an examination executor is able to perform an examination on a clinical position corresponding to the attention area while checking the attention area on the breast image.

In an ultrasound examination, a body mark which indicates right or left breast and an inspection position is recorded in an ultrasound image. If the body mark is recorded incorrectly due to a busy schedule or lack of concentration, there may be some confusion for a doctor who makes a diagnosis while observing the ultrasound image with reference to the recorded body mark. However, the estimated clinical position information displayed in step S8 appears on the frontal schema image which has the same form or configuration as a body mark. Therefore, when an ultrasound examination has been performed on a position corresponding to the estimated clinical position information, an examination executor has only to transfer the estimated clinical position information to the ultrasound image as a body mark. This prevents recording wrong information in an ultrasound image.

When there is another attention area to be subsequently examined after an ultrasound diagnosis has been made on the basis of estimated clinical position information, an examination executor is able to select, through the operation part 42, a different attention area from a breast image displayed on the viewer screen 431. When a different attention area is selected, the control part 41 of the image display device 4 transmits positional information on the selected attention area to the server device 3 through the communication part 44. When there is no attention area to be subsequently examined, an examination executor carries out an operation to move to a different screen or to close the screen, through the operation part 42. In this case, the control part 41 transmits an instruction to terminate the processing for producing estimated clinical position information to the server device 3 through the communication part 44.

The control part 31 of the server device 3 determines, after displaying the estimated clinical position information, whether or not a positional information on the subsequent attention area has been received from the image display device 4 through the communication part 34 (step S9). When the control part 31 determines that the positional information on the subsequent attention area has been received from the image display device 4 through the communication part 34 (Yes in step S9), the control part 31 returns to step S5 in the processing, and executes the processing between step S5 and step S9 repeatedly. When the control part 31 determines that the positional information on the subsequent attention area has not been received but instead an instruction to terminate the processing has been received from the image display device 4 through the communication part 34 (No in step S9 and Yes in step S10), the control part 31 terminates the processing for producing the estimated clinical position information.

As has been previously described, in the medical imaging system 100, the control part 31 of the server device 3, when an attention area is specified on a breast image in the image display device 4, estimates a position of the attention area in a breast as viewed from the front, on the basis of the position of the attention area in the breast image and an angle at which an X-ray photographing device is inclined at the time of taking the breast image. Then the control part 31 produces estimated clinical position information which indicates the estimated position of the attention area. After that, the control part 31 causes the display part 43 of the image display device 4 to display the produced estimated clinical position information.

As a consequence, an examination executor of an ultrasound examination is easily able to identify a position in a breast corresponding to an attention area on a breast image, when a mammography examination and the ultrasound examination are performed as separate examinations.

Specifically, the control part 31 places an MLO image in an inclined manner with respect to a frontal schema image, on the basis of an angle at which the image production device 1 is inclined at the time of taking the MLO image, as well as places an CC image on the basis of an angle of the image production device 1 at the time of taking the CC image. Then the control part 31 projects an attention area in the MLO image and an attention area in the CC image on the frontal schema image, so that an area in which the projected attention areas overlap can be estimated to be a position of the attention area in an imaging-object region as viewed from the front.

Further, for example, the frontal schema image is divided into a plurality of segments. Then the control part 31 calculates the size of an area occupied by an estimated position of the attention area in each of the segments of the frontal schema image, so that information indicating which of the segments includes the area of the largest calculated size can be produced as estimated clinical position information.

Furthermore, as a result of displaying a breast image on which a specified attention area is indicated and estimated clinical position information on the same screen, an examination executor of an ultrasound examination is able to perform the ultrasound examination while checking the attention area on a radiographic image of the breast.

Moreover, it becomes easier to specify an attention area, by adding an annotation to an area of an abnormal shadow candidate on a radiographic image of a breast, on the viewer screen 431 on which an attention area is to be specified.

The description of the foregoing embodiment includes a preferred example of the present invention, but is not limited to the above example.

For example, in the above embodiment, the server device 3 and the image display device 4 are separate devices to form a client-server system. Alternatively, functions of the two devices may be fulfilled by a single device. Specifically, a storage part of the single device stores a program to cause a computer to function as a display means, a specification means, an estimation means, a production means, and a display control means, and functions of these means are fulfilled in cooperation between a control part such as a CPU and the program. In addition, a function of the abnormal shadow candidate detection device 2 may be incorporated into the same single device.

In the above embodiment, a position of an abnormal shadow candidate detected by the abnormal shadow candidate detection device 2 is indicated on the viewer screen 431, for the purpose of assisting an examination executor in specifying an attention area. Alternatively, a position of an abnormal shadow candidate may be manually input by a radiological technologist or the like through an input means, so as to be shown on the screen. For example, in an image display device 4 placed in a radiology department, a radiological technologist enters (specifies) an abnormal shadow candidate, through the operation part 42, on a breast image displayed on the viewer screen 431. Then abnormal shadow candidate information on the specified abnormal shadow candidate is linked with the breast image in which the abnormal shadow candidate has been detected, and the information is stored in the image DB 351. The abnormal shadow candidate information is displayed on the breast image, according to operation through the operation part 42 (for example, the abnormal shadow candidate information is displayed in response to operation of a CAD button 431e by an examination executor of an ultrasound examination). In this way, when a radiological technologist or the like has determined, as a result of observation, that an abnormal shadow candidate exists in a certain area, an examination executor of an ultrasound examination is easily able to locate the area and perform the examination thereon.

In the above embodiment, a description has been provided of a case where an imaging-object region is a breast, as an example. However, the present invention is applicable in assisting an ultrasound diagnosis on a different region.

In the above embodiment, a description has been provided of a case where clinical position information is displayed for an ultrasound diagnosis. However, the present invention is applicable as a training tool for an examination executor who makes an ultrasound diagnosis.

Moreover, in the preceding description, there has been disclosed an example in which a hard disk drive, a semiconductor non-volatile memory, or the like is used as a computer-readable storage medium storing a program according to an aspect of the present invention. However, the medium is not limited to this example. A portable recording medium such as a compact disk read only memory (CD-ROM) is applicable as another option of the computer-readable storage medium. Also, a carrier wave is applicable as a medium to provide data of the program according to an aspect of the present invention through communication lines.

Additionally, modifications and alterations may be made as appropriate in details of configurations and functions of components forming the above medical imaging system, without departing from the spirit of the present invention.

Although the present invention has been described and illustrated in detail, it is clearly understood that the same is by way of illustrated and example only and is not to be taken by way of limitation, the scope of the present invention being interpreted by terms of the appended claims.

## Claims

1. A medical imaging system (100) **characterized by** comprising:
a display means configured to display at least one medical image acquired by X-ray photographing an imaging-object region;
a specification means configured to specify an attention area in the medical image displayed by the display means;
an estimation means configured to estimate a position of the attention area in the imaging-object region as viewed from the front, on the basis of a position of the attention area in the medical image and an inclination angle of an X-ray photographing device at the time of capturing the medical image;
a production means configured to produce estimated clinical position information indicating the position of the attention area in the imaging-object region as viewed from the front, the position having been estimated by the estimation means; and
a display control means configured to cause the display means to display the produced estimated clinical position information.

2. The medical imaging system (100) according to claim 1,
**characterized in that** the estimation means estimates the position of the attention area in the imaging-object region as viewed from the front, by placing the medical image in an inclined manner with respect to a frontal image of the imaging-object region on the basis of the inclination angle of the X-ray photographing device, and by projecting the attention area in the medical image on the frontal image of the imaging-object region, and
the production means produces the estimated clinical position information on the basis of the frontal image of the imaging-object region on which the attention area has been projected.

3. The medical imaging system (100) according to claim 2,
**characterized in that** the frontal image of the imaging-object region is divided into a plurality of segments, and
the production means calculates the size of an area occupied by the estimated position of the attention area in each of the segments, and produces information indicating which of the segments includes the area of the largest calculated size, the information being produced as the estimated clinical position information.

4. The medical imaging system (100) according to any one of claims 1 to 3,
**characterized in that** the imaging-object region is a breast, and
the medical image is a breast image acquired by X-ray photographing the breast.

5. The medical imaging system (100) according to claim 4,
**characterized in that** the breast image includes an MLO image captured by photographing a breast as the imaging-object region from an oblique direction, and a CC image captured by photographing the breast from a vertical direction, and
the estimation means places the MLO image in an inclined manner with respect to the frontal image of the imaging-object region on the basis of an inclination angle of the X-ray photographing device at the time of capturing the MLO image, as well as places the CC image on the basis of an angle of the X-ray photographing device at the time of capturing the CC image, projects the attention area in the MLO image and the attention area in the CC image on the frontal image of the imaging-object region, and estimates that an area in which projected images of the attention area overlap is a position of the attention area in the imaging-object region as viewed from the front.

6. The medical imaging system (100) according to any one of claims 1 to 5,
**characterized in that** the display control means displays the estimated clinical position information on the same screen as the medical image on which the attention area is shown.

7. The medical imaging system (100) according to any one of claims 1 to 6, **characterized by** further comprising a detection means configured to detect an abnormal shadow candidate in the medical image,
**characterized in that** the display means displays an area of the abnormal shadow candidate detected by the detection means on the medical image.

8. The medical imaging system (100) according to any one of claims 1 to 6, **characterized by** further comprising an input means configured to input an area of an abnormal shadow candidate through the medical image,
**characterized in that** the display means displays the area of the abnormal shadow candidate input by the input means on the medical image.

9. A program for causing a computer to function as:
a display means configured to display at least one medical image acquired by X-ray photographing an imaging-object region;
a specification means configured to specify an attention area in the medical image displayed by the display means;
an estimation means configured to estimate a position of the attention area in the imaging-object region as viewed from the front, on the basis of a position of the attention area in the medical image and an inclination angle of an X-ray photographing device at the time of capturing the medical image;
a production means configured to produce estimated clinical position information indicating the position of the attention area in the imaging-object region as viewed from the front, the position having been estimated by the estimation means; and
a display control means configured to cause the display means to display the produced estimated clinical position information.
